# EUROPEAN PATENT SPECIFICATION

(11) **EP 4 427 723 B1**
(45) Date of publication and mention of the grant of the patent: **26.08.2026**
(21) Application number: 22886784.2
(22) Date of filing: 18.10.2022
(51) Int. Cl.: A61K 6/887, A61K 6/15, A61K 6/60, A61K 6/64, A61K 6/65, A61K 6/76, A61C 13/00

(54) **HYBRID RESIN-BASED BLANK FOR DENTAL CUTTING, AND METHOD FOR MANUFACTURING CROWN**
HYBRIDER HARZBASIERTER ROHLING ZUM ZAHNSCHNEIDEN UND VERFAHREN ZUR HERSTELLUNG EINER KRONE
ÉBAUCHE À BASE DE RÉSINE HYBRIDE POUR DÉCOUPE DENTAIRE, ET PROCÉDÉ DE FABRICATION DE COURONNE

(30) Priority: 01.11.2021 JP 2021178902
(43) Date of publication of application: 11.09.2024
(73) Proprietor: Tokuyama Dental Corporation, Tokyo 110-0016 (JP)
(72) Inventor: YAMANE Shintaro, Tokyo 110-0016 (JP); NAGASAWA Yuukou, Tokyo 110-0016 (JP)
(74) Representative: HGF
(86) International application number: PCT/JP2022/038673
(87) International publication number: WO 2023/074456

(56) References cited:
- WO-A1-2019/189698
- WO-A1-2019/189698
- WO-A1-2020/050123
- WO-A1-2021/131490
- JP-A- 2014 000 176
- JP-A- 2017 213 394
- JP-A- 2018 095 620

## Description

### TECHNICAL FIELD

The present invention relates to a hybrid resin blank for dental milling and to a method for fabricating a crown using such a blank.

### BACKGROUND ART

Dental restorations (dental prostheses), such as inlays, onlays, crowns, bridges, and implant superstructures, for dental treatment have come to be frequently fabricated using CAD/CAM systems, which use techniques including intraoral imaging, computer aided design (CAD), and computer aided manufacturing (CAM) to perform milling on blanks for dental milling made of non-metallic material to form dental restorations. As used herein, the term "a blank for dental milling" means a workpiece (also referred to as a "mill blank") configured to be attachable to a milling machine in a CAD/CAM system, generally known examples of which include rectangular or cylindrical (solid) blocks, (solid) plates or disks, and so on. In this regard, fixing pins for fixation to milling machines are often joined to blanks for dental milling, and blanks integrated with such fixing pins may also be referred to as blanks for dental milling. Thus, the term "a blank for dental milling" is intended to also include such fixing pin-integrated blanks. Moreover, its main body to be subjected to milling (the main body of the blank for dental milling) will also be referred to as the "workpiece portion".

For high workability (milling workability), high esthetic quality, and high strength, many blanks for dental milling are hybrid resin blanks (hereinafter also referred to as "HR blanks"), which have a block-shaped workpiece portion made of a resin material resulting from curing of a curable composition including an inorganic filler, such as silica, a polymerizable monomer, such as a methacrylate compound, and a polymerization initiator (such a resin material will also be referred to as "hybrid resin (HR)").

Meanwhile, highly esthetic restoration requires determining the color (defined by hue, lightness, and chroma, in other words hue and color tone) of the tooth to be restored (restoration target tooth) or teeth around the restoration target tooth and selecting a HR blank having a workpiece portion with a color matching with the determined color. In this regard, such color determination is also called "shade taking". Shade taking is usually performed using tooth color samples called shade guides. A variety of shade guides exist, which are devised in terms of the number of color samples and the structure of the color sample holder. Among them, shade guides under the trade name of "VITA Classical" manufactured by VITA (also referred to as "VITA shade guides") are in most widespread use (see, for example, Patent Document 1). A VITA shade guide includes samples with 16 colors in total (hereinafter, a color identified using the index of a combination of hue and a mixture of lightness and chroma or using the index based on hue, lightness, and chroma is also referred to as "shade"). The colors of such a shade guide can be compared with the color of the target to be restored and the color of teeth around the target for the determination of the color of the target. In VITA shade guides, shades A (reddish brown), B (reddish yellow), C (gray), and D (reddish gray) are classified according to lightness and represented by symbols. The 16 shades include B1, A1, B2, D2, A2, C1, C2, D4, A3, D3, B3, A3.5, B4, C3, A4, and C4 in descending order of lightness.

HR blanks include a single-color product having a workpiece portion with a single HR layer and a multi-color product having a workpiece portion with HR layers with different colors (see, for example, Patent Document 2). Both of such products are often available in the 16 shades or some of them selected as basic shades (only in the number of basic shades). Specifically, workpiece portions of HR blanks have the following surface color arrangement patterns. The monolayer-structure HR blank has a workpiece portion with its entire surface in a single specific basic shade (color) while the multilayer-structure HR blank has a layer with a specific basic shade and another layer with a shade other than the basic shade and/or a color other than the 16 shades, which is thus colored with different colors depending on the multilayer structure.

A known monolayer-structure HR blank also has what is called a structural color, which can match with the color of natural teeth without addition of any pigment or colorant (hereinafter, such a blank will also be referred to as a "structural color HR blank"). For example, Patent Document 3 discloses a resin block for dental milling, including a resin matrix (A) and a spherical filler (B) with an average particle diameter in a range of 230 nm to 1,000 nm, wherein when 10 mm thick, the resin block has a lightness (V) of less than 5.0 and a chroma (C) of less than 2.0 for colored light in Munsell color system as measured on a black background and a white background with a colorimeter, and when 1 mm thick, the resin block has a lightness (V) of less than 5.0 and a chroma (C) of 0.05 or more for colored light in Munsell color system as measured on a black background with a colorimeter, and has a lightness (V) of 6.0 or more and a chroma (C) of less than 2.0 for colored light in Munsell color system as measured on a white background with a colorimeter.

Patent Document 3 suggests that when 90% or more of the individual particles in the spherical filler (B) have particle diameters falling within the range of ±5% of the average particle diameter and the resin matrix (A) and the spherical filler (B) satisfy the condition nP < nF, wherein nP is the refractive index of the resin matrix (A) at 25°C, and nF is the refractive index of the spherical filler (B) at 25°C, the resin block can have a structural color with a specific tone depending on the particle diameter of the spherical filler (B), can have such properties that its specific reflectance spectrum is clearly observable on a black background depending on its color while it exhibits, on a white background, a substantially constant level of reflectance over substantially the whole of the visible spectrum with no observable visible spectral light, and has the effect of widely matching with various surroundings in particular when it exhibits (develops) a yellowish to reddish structural color.

The workpiece portion of a structural color HR blank includes a HR having a structural color (hereinafter, also referred to as a "structural color HR"). It is known that a composite material including a resin matrix and inorganic particles dispersed in the matrix (such a composite material corresponds to a structural color HR) can exhibit a structural color with a constant tone (hereinafter such a structural color will also be referred to as a "specific structural color") regardless of changes in incident light angle when the shape and size distribution of the inorganic particles, the relationship between the refractive indices of the inorganic particles and the resin matrix, and the dispersion state of the inorganic particles satisfy specific conditions (see, for example, Patent Document 4).

Besides the specific structural color, known structural colors used in the dental field also include those known as so-called opalescent effect (see, for example, Patent Document 5), and a HR blank including: an HR having an opalescent effect; and a colorant blended in the HR to provide a specific shade to the HR is also known.

### Citation List

### Patent Document

Patent Document 1: Japanese Patent No. 6004769
Patent Document 2: Japanese Unexamined Patent Application, Publication No. 2017-213394
Patent Document 3: PCT International Publication No. WO2019/189698
Patent Document 4: PCT International Publication No. WO2020/050123
Patent Document 5: PCT International Publication No. WO2011/158742

### DISCLOSURE OF THE INVENTION

### Problems to be Solved by the Invention

Meanwhile, some dental restorations are prepared using a HR blank with a specific shade adjusted with a colorant, and such a HR blank usually has a color (shade) selected by shade taking based on the appearance color of the HR blank prior to the milling.

Unfortunately, when a crown (a prosthesis for covering an anchor tooth) is prepared with such a HR blank with a specific shade adjusted with a colorant, its appearance color (shade) may change, and as a result, the prepared crown may fail to provide desired color tone compatibility when attached to the anchor tooth (which is shaped by milling and shaping the target tooth so as to have a shape matching with that of the crown).

Patent Document 3 suggests that a dental prosthesis prepared from a structural color HR blank can provide a restoration matching with the color tone of natural teeth. Unfortunately, Patent Document 3 shows color tone compatibility evaluation performed only on a dental prosthesis (inlay) fitting into a class II cavity (5 mm in diameter and 3 mm in depth) formed in an artificial tooth at lower right No. 6 (first molar) and does not show any color tone compatibility for crowns. With a concern about the possibility of the above problem with crowns prepared with structural color HR blanks, the inventors have made an investigation on the color tone compatibility of crowns prepared with structural color HR blanks. As a result, the inventors have found that the same problem occurs on such crowns.

Thus, it is a first object of the present invention to reduce changes in shade occurring in the preparation of crowns with HR blanks with a specific shade adjusted using a colorant. It is a second object of the present invention to provide a HR blank that can exhibit a specific structural color as disclosed in Patent Document 3, can maintain high color tone compatibility when formed into an inlay, and can provide good color tone compatibility even when formed into a crown and subjected to attachment.

### Means for Solving the Problems

The invention is defined by the independent claims 1 and 4. A first aspect of the present invention is directed to a hybrid resin blank (HR blank) to be subjected to dental milling, the HR blank including: a monolayer- or multilayer-structure workpiece portion including a layer of a colorant-containing structural color hybrid resin (colorant-containing structural color HR) including: a resin matrix of a structural color hybrid resin (structural color HR) containing dispersed inorganic particles and exhibiting a structural color with a specific color tone; and a colorant including one or more coloring materials and dispersed in the resin matrix, the colorant-containing structural color HR containing 800 to 8,000 ppm by mass of the colorant based on the total mass of the colorant-containing structural color HR, the colorant including 70 mass% or more of a similarly chromatic coloring material, as determined by the following manner: in a spectral reflectance curve of the colorant-free structural color hybrid resin obtained by measuring a 3 mm-thick sample of the colorant-free structural color hybrid resin on a black background with a colorimeter, in which a horizontal axis represents reflected light wavelength (nm) and a vertical axis represents reflectance (%), a wavelength at which a maximum reflectance derived from the structural color occurs is defined as a structural color wavelength and a wavelength region in which 85% or more of the maximum reflectance occurs is defined as a wavelength region A; and in spectral reflectance curve of a coloring material, of the one or more coloring materials, obtained by measurement on a black background with a colorimeter, a wavelength at which a maximum reflectance occurs is defined as a coloring wavelength of the coloring material, a coloring material, of the one or more coloring materials, whose coloring wavelength exists in the wavelength region A is defined as a similarly chromatic coloring material, and a coloring material, of the one or more coloring materials, whose coloring wavelength exists outside the wavelength region A is defined as a differently chromatic coloring material.

The HR blank of the present invention has a structural color wavelength in the range of 590 to 690 nm. When 1 mm thick, the colorant-containing structural color HR has a spectral reflectance curve with a first maximum reflectance ML (%) and a second maximum reflectance MH (%) satisfying the condition: MH/ML (the ratio of MH to ML) > 1.00 as measured on a black background with a colorimeter, wherein the ML is the maximum reflectance in the wavelength range of 400 to 500 nm of the spectral reflectance curve, and the MH is the maximum reflectance in the wavelength range of 600 to 750 nm of the spectral reflectance curve.

The HR blank of the present invention preferably has a workpiece portion having (1) a monolayer structure including the colorant-containing structural color HR or (2) a multilayer structure including two or three layers including: a first layer that is provided at one end of the multilayer structure and includes the colorant-containing structural color HR; and a second layer that includes another colorant-containing structural color HR having an appearance color different from that of the colorant-containing structural color HR, a colorant-free structural color HR, or a HR other than these HRs.

A second aspect of the present invention is directed to a method for fabricating a crown, the method including: milling the workpiece portion of the HR blank of the first aspect of the invention by a CAD/CAM technique based on intraoral three-dimensional shape data on a patient having a shaped anchor tooth to fabricate a crown to be attached to the anchor tooth.

### Effects of the Invention

The HR blank of the present invention less changes its appearance color before and after being milled into a crown. Thus, the HR blank of the present invention can be formed into a crown with desired good color tone compatibility after shade taking based on the appearance color of the HR blank for milling.

### PREFERRED MODE FOR CARRYING OUT THE INVENTION

The inventors have first conducted a study on what can cause the problem of failing to achieve desired color tone compatibility, which occurs when a conventional common HR blank having a workpiece portion including HR having a specific shade adjusted with a colorant is milled into a crown and the resulting crown is subjected to practical fitting. As a result, it has been found that such a blank will significantly change its appearance color before and after being milled into a particularly thin crown because the crown (a dental prosthesis for covering the anchor tooth), which has an anchor tooth engaging cavity, is formed with a thickness varying depending on the size and shape of the cavity (corresponding to the size and shape of the anchor tooth).

The cause of difficulty in achieving good color tone compatibility for the crown prepared using a structural color HR blank may be as follows. An inlay, which has faces (side and bottom faces) joined to natural teeth except for its exposed upper face, can exhibit good color tone compatibility since the light determining its appearance color includes not only structural color-reflecting light but also a large amount of light passing through it, which reflects the color of the natural teeth around it. On the other hand, the crown contacts with the natural tooth only in its inside and thus can hardly have such an effect as produced by the inlay.

Conventional common HR blanks, which have a certain degree of transparency and a constant colorant content (which cannot be changed ex-post), will inevitably change their appearance color depending on their thicknesses. In this regard, the effect of thickness on color tone and the effect of the size of the exposed surface can be reduced using a combination of a structural color HR blank, which has a structural color (regardless of colorants), and a colorant in an amount that will not hinder the color tone compatibility produced by the structural color, which may lead to a solution to the first and second problems described above. Based on such an idea, a study has been conducted on a blank system including a structural color HR and a colorant. As a result, the following has been found. With a low colorant content, the blank can have high transparency, and thus, when milled into a thickness of 2 mm or less, it can significantly change its appearance color due to the color of the base that can be seen through it. On the other hand, when the color of the blank is adjusted using a relatively large amount of a colorant with a color of the same type as that of the structural color, the thickness-dependent changes in appearance color can be reduced.

Specifically, the workpiece portion according to the present invention includes a structural color HR and a controlled type and amount of a colorant(s), which can reduce thickness-dependent changes in shade within a certain range and thus provide a solution to the first problem. Moreover, the structural color HR blank contains a colorant(s) so as to satisfy a specific condition, which can provide a solution to the second problem.

The structural color HR blank containing a relatively large amount of a colorant may have a slightly higher risk of discoloration during long-term use and needs to be prepared in several shades (with different colorant contents) to address a number of shades, which leads to the need for shade taking before use. However, the structural color HR blank is significantly advantageous in that it can be adapted as a single product for both inlays and crowns. Moreover, even when formed into an inlay, the structural color HR blank can still make highly esthetic restoration possible because it still has a certain degree of toning effect produced by harmonization between structural color and surrounding color. A colorant-free structural color HR blank may have an appearance color different from the practical shade (of the patient tooth), which may make the user feel somewhat unsatisfied. However, the structural color HR blank is secondarily advantageous in that it can sweep away such a concern.

Although not bound by any particular theory, the inventors have the following speculation about the cause of the above problem, which can be significant for a mixture of a structural color HR and a colorant, and about the reason for which the problem can be prevented when the color is adjusted using a relatively large amount of a colorant with a color of the same type as that of the structural color. The structural color and the color of a colorant, which have different coloring mechanisms, are differently influenced by thickness. Thus, if a large amount of a colorant with a color of a type different from the structural color is added to the structural color HR, the thickness change by milling will break the coloring balance to significantly change the appearance color. However, when a colorant with a color of the same type as the structural color is added to the structural color HR, the thickness change by milling and the resulting broken balance between the structural color and the color of the colorant will have no significant influence on the appearance color, and the structural color can exhibit a broad peak over a relatively wide wavelength range in the resulting spectral reflectance curve.

Hereinafter, the HR blank of the present invention and the method for fabricating a crown using the HR blank will be described in detail.

As used herein, the expression "x to y", in which x and y are numerical values, means "x or more and y or less" unless otherwise stated. When a unit is attached only after the value y for the expression, such a unit is intended to be attached also after the value x. Unless otherwise specified, the term "refractive index" means the refractive index measured at 25°C using sodium D-line (589 nm in wavelength).

### Outline of the HR Blank of the Present Invention

The workpiece portion of the HR blank of the present invention includes a structural color HR and a colorant(s) satisfying a specific condition and thus has a specific shade. Thus, the HR blank of the present invention has a significant feature in that its appearance color will maintain the original shade even when it is formed into a crown. The HR blank of the present invention also provides a solution to a special problem with a conventional structural color HR blank having a layer of a structural color HR as disclosed in Patent Document 4 or 5. The HR blank of the present invention has the following features: (1) It contains a specific amount of a colorant for color control, which can prevent changes in appearance color due to the color of a base, such as an anchor tooth, which would otherwise be seen through it, when it is formed into a crown; (2) The content of a certain coloring material(s) with a color of the same type as the structural color in the colorant falls within a specific range, which can reduce thickness-dependent changes in appearance color.

Specifically, the HR blank of the present invention has a monolayer- or multilayer-structure workpiece portion including a layer of a colorant-containing structural color HR including: a resin matrix of a structural color HR containing dispersed inorganic particles and exhibiting a structural color with a specific color tone; and a colorant including one or more coloring materials and dispersed in the resin matrix, in which the colorant-containing structural color HR contains 800 to 8,000 ppm by mass of the colorant based on the total mass of the colorant-containing structural color HR, and the colorant includes 70 mass% or more of a similarly chromatic coloring material as determined by the following manner: in a spectral reflectance curve of the colorant-free structural color hybrid resin obtained by measuring a 3 mm-thick sample of the colorant-free structural color hybrid resin on a black background with a colorimeter, in which a horizontal axis represents reflected light wavelength (nm) and a vertical axis represents reflectance (%), a wavelength at which a maximum reflectance derived from the structural color occurs is defined as a structural color wavelength and a wavelength region in which 85% or more of the maximum reflectance occurs is defined as a wavelength region A; and in spectral reflectance curve of a coloring material, of the one or more coloring materials, obtained by measurement on a black background with a colorimeter, a wavelength at which a maximum reflectance occurs is defined as a coloring wavelength of the coloring material, a coloring material, of the one or more coloring materials, whose coloring wavelength exists in the wavelength region A is defined as a similarly chromatic coloring material, and a coloring material, of the one or more coloring materials, whose coloring wavelength exists outside the wavelength region A is defined as a differently chromatic coloring material.

In this regard, the "colorant-free structural color HR" corresponds to the composite material disclosed in Patent Documents 3 to 5 (being colorant-free and for dental use) and specifically corresponds to a product resulting from the curing of a raw material composition (polymerizable and curable) including all materials for the production of the "colorant-containing structural color HR" except for the colorant(s). The measurement for determining the structural color wavelength specifically includes measuring the spectral reflectance of a 3 mm-thick sample (e.g., a sample polished to gloss), which is made of a product resulting from the curing of the raw material composition, on a black background using a spectrophotometer (e.g., TC-1800MKII manufactured by Tokyo Denshoku Co., Ltd., halogen lamp: 12 V, 100 W; measurement wavelength range: 380 to 780 nm). The resulting spectral reflectance curve has a horizontal axis representing reflected light wavelength (nm) and a vertical axis representing reflectance (%). In the spectral reflectance curve, the wavelength at which the maximum reflectance occurs may be determined to be the structural color wavelength, and the wavelength region in which 85% or more of the maximum reflectance occurs may be determined to be the wavelength region A. The wavelength region A is usually between a wavelength being shorter than the structural color wavelength and providing 85% of the maximum reflectance and another wavelength being longer than the structural color wavelength and providing 85% of the maximum reflectance.

The coloring wavelength of the colorant is determined as follows. Similar to the above, the spectral reflectance of a 3 mm-thick HR blank sample (not exhibiting any structural color) resulting from the curing of a composition containing 2 to 10 mass% of the colorant based on the total mass of the composition is measured on a black background. In the resulting spectral reflectance curve, the wavelength at which the maximum reflectance occurs can be determined to be the coloring wavelength of the colorant.

In some cases, the reflectance peak (or pattern) derived from the colorant is known and distinguishable from the reflectance peak (or pattern) derived from the structural color. In such cases, the structural color wavelength and the wavelength region A may be determined based on the spectral reflectance curve resulting from the spectral reflectance measurement of a 3 mm-thick sample of the colorant-containing structural color HR on a black background.

The HR blank of the present invention is not particularly different from the conventional HR blank except for the features described above. If necessary, the HR blank of the present invention may have a fixing member, such as a fixing pin, for fixing it to a milling machine. The workpiece portion may have any shape and any size, such as a rectangle- or cylinder-shaped (solid) block or a plate- or disk-shaped (solid) disk.

In a case where the workpiece portion has a multilayer structure including a layer of the colorant-containing structural color HR, it may include a layer of a colorant-free structural color HR or a layer of a non-structural-color HR. For example, the workpiece portion preferably has a multilayer structure including two or three layers including: a first layer that is provided at one end of the multilayer structure and includes the colorant-containing structural color HR; and a second layer that includes another colorant-containing structural color HR having an appearance color different from that of the colorant-containing structural color HR, a colorant-free structural color HR, or a HR other than these HR.

As mentioned above, HR blanks are often available in 16 shades, which are broadly classified into shades A (reddish brown), B (reddish yellow), C (gray), and D (reddish gray), or in several basic shades selected from the 16 shades. For ease of adjusting the color to each shade, the structural color wavelength of the HR blank is in the range of 590 to 690 nm. Moreover, for the suppression of changes in appearance color depending on the thickness after the milling in the case of adjusting the color to such a shade, the colorant-containing structural color HR, when 1 mm thick, has a spectral reflectance curve with a first maximum reflectance ML (%) and a second maximum reflectance MH (%) satisfying the condition: MH/ML (the ratio of MH to ML) > 1.00 as measured on a black background with a colorimeter, in which the ML is the maximum reflectance in the wavelength range of 400 to 500 nm of the spectral reflectance curve, and the MH is the maximum reflectance in the wavelength range of 600 to 750 nm of the spectral reflectance curve.

As mentioned above, some structural color HRs produce an opalescent effect as shown in Patent Document 5. Such structural color HRs are often used to form materials that can exhibit some transparency with a low colorant content and can reproduce the enamel appearance of a cut-end portion of a tooth. To form a blank for dental milling, such a structural color HR is disposed at the distal end portion of a multilayer structure to provide a cut-end portion for a crown resulting from working. In such a case, the base-seen-through problem can hardly occur, and thickness-dependent changes in appearance color can also hardly occur (due to a relatively high degree of transparency). On the other hand, the structural color HR exhibiting a specific structural color as disclosed in Patent Document 4, specifically a structural color HR exhibiting (developing) a specific yellowish to reddish structural color can easily suffer from the problem described above and thus can be highly effectively improved by the present invention because such a structural color HR sometimes should contain a large amount of a colorant for adjusting the shade as shown above and should form a crown material for forming a region from the center to neck of the crown and for reproducing the color of the dentin.

Hereinafter, therefore, the HR blank of the present invention based on a structural color HR will be described after a brief description of the structural color HR (mainly the colorant-free structural color HR exhibiting a specific structural color) and the raw materials therefor.

### Structural Color HR

Patent Document 4 shows conditions for a composite material (corresponding to HR) to exhibit a structural color with a constant color tone (specific structural color) regardless of changes in incident light angle due to optical interference and so on. According to the document, conditions I to V below should be satisfied.

Condition I: Inorganic particles dispersed in the resin matrix of a cured product should include one or more groups of identical diameter spherical particles (G-PID).

The term "G-PID" refers to a group of spherical inorganic particles having a specific average primary particle diameter in the range of 100 to 1,000 nm, in which individual spherical inorganic particles are made of substantially the same material and which has a number-based particle size distribution with 90% or more of all particles falling within the range of ±5% of the specific average primary diameter. The term "the average primary particle diameter of spherical inorganic particles" means the average of diameters (maximum diameters) of 30 or more primary particles selected and observed in a unit field of view of a G-PID photograph taken with a scanning electron microscope. The term "spherical" is intended to include "substantially spherical shapes" and should not be construed as limiting to perfect spheres. Specifically, spherical particles may include particles with an average uniformity of 0.6 or more, more preferably 0.8 or more, as determined by a process including: measuring the maximum diameters of 30 or more particles observed in a unit field of view of a G-PID photograph taken with a scanning electron microscope; dividing, by the maximum diameters, the particle diameters perpendicular to the maximum diameters; and averaging the resulting quotients.

In this regard, Patent Document 4 discloses a structural color HR that exhibits a structural color with a color tone depending on the average primary particle diameter of G-PID and suggests that the HR should contain G-PID with an average primary particle diameter of 230 to 800 nm in order to exhibit a yellowish to reddish structural color, preferably a specific structural color with a wavelength in the range of 590 to 690 nm.

Condition II: When the inorganic particles include a number a of G-PIDs represented by G-PIDₘ in ascending order of the average primary particle diameter, wherein m is 1 when a is 1 and m is a natural number of 1 to a when a is 2 or more, the material constituting the individual particles may differ between the series of G-PIDₘ (when a is 2 or more), and in such a case, the average primary particle diameters of the series of G-PIDₘ should differ by 25 nm or more from one another.

Condition III: The refractive index n_{(MX)} of the resin matrix and the refractive index n_{(G-PIDm)} of the spherical inorganic particles in each of the series of G-PIDₘ should satisfy the relation: ${n}_{\left(MX\right)} < {n}_{\left(G-PIDm\right)}$ for each n_{(G-PIDm)}.

In this regard, in view of the visibility and clarity of the structural color, the difference Δn between n_{(G-PIDm)} and n_{(MX)} (= n_{(G-PIDm)} - n_{(MX)}) is preferably 0.001 to 0.1, more preferably 0.002 to 0.1, even more preferably 0.005 to 0.05, for each n_{(G-PIDm)}. The refractive index n _{(MX)} of the resin matrix is, for example, in the range of 1.40 to 1.57, and the refractive index n_{(G-PIDm)} of the spherical inorganic particles is, for example, in the range of 1.45 to 1.58.

Condition IV: The structure of arrangement of the spherical inorganic particles in all G-PIDs in the resin matrix should have a short-range order structure satisfying conditions 1 and 2 below with respect to the radial distribution function g(r) defined by Formula (1): g(r) = {1/<ρ>} × {dn/da}, which is a function representing the probability of occurrence of a spherical inorganic particle at a point distant by a distance r from the center of any other spherical inorganic particle dispersed in the composite material, in which <ρ> is the average density of spherical inorganic particles in an observation plane determined from a scanning electron micrograph of an interior of the composite material, dn is the number of spherical inorganic particles occurring in the region between a circle with a distance r from any spherical inorganic inorganic particle and another circle with a distance r + dr in the observation plane, and da is the area of the region, provided that da = 2πr·dr.

Condition 1: The distance r₁ between nearest neighbor particles should be one time or more and two times or less the average particle diameter r₀ of all spherical inorganic particles dispersed in the composite material, in which r₁ is defined as r corresponding to the top of a peak closest to the origin point among the peaks occurring in a radial distribution function graph showing the relationship between r/r₀, which is a normalized dimensionless number obtained by dividing, by r₀, the distance r from the center of any spherical inorganic particle dispersed in the composite material and represented by the x axis of the graph, and g(r) corresponding to r, which is represented by the y axis of the graph.

Condition 2: The radial distribution function g(r) should have a minimal value of 0.56 or more and 1.10 or less between the nearest neighbor particle distance r₀ and the second-nearest-neighbor particle distance r₂, which is defined as r corresponding to the top of a peak second-closest to the origin point among the peaks occurring in the radial distribution function graph.

Condition V: The resin matrix may contain a group of superfine inorganic particles (G-SFP) with an average primary particle diameter of less than 100 nm. In such a case, the average primary particle diameter of the G-SFP should be 25 nm or more smaller than the average primary particle diameter of G-PID₁.

As used herein, the term "the average primary particle diameter of the inorganic particles" means the average of diameters (maximum diameters) of 30 or more primary particles observed in a unit field of view of a G-SFP photograph taken with a scanning electron microscope.

### Raw Material Composition for Structural Color HR

Patent Document 4 discloses that the cured product (a composite material corresponding to HR) satisfying conditions I to V can be produced by curing a curable composition including: 100 parts by mass of a polymerizable monomer(s); 10 to 1,500 parts by mass of one or more groups of identical diameter spherical particles (G-PID); and 0.01 to 10 parts by mass of a polymerization initiator, and optionally 0.1 to 50 parts by mass of a group of superfine particles (G-SFP), which is prepared by the mixing method (a) or (b) shown below.
(a) A method including: preliminarily performing mixing under different mixing conditions to form curable compositions having the same or substantially the same composition as the curable composition to be produced practically; examining the radial distribution function g(r) on the products resulting from the curing of the resulting mixtures obtained under the different mixing conditions to determine the mixing condition satisfying conditions 1 and 2; and performing mixing under the same condition as the determined mixing condition.
(b) A method including: performing mixing; sampling some of the resulting mixture during the mixing step and/or after the completion of the mixing step; curing the sampled mixture to form a cured product; determining whether or not the dispersion state of the inorganic particles in the cured product satisfies conditions 1 and 2; and continuing the mixing until conditions 1 and 2 are satisfied.

Hereinafter, a colorant-free curable composition that is prepared by such a method and capable of producing a cured product satisfying conditions I to V is also referred to as the "colorant-free raw material composition for specific structural color HR".

Patent Document 4 also states that for ease of obtaining the cured product (composite material) having the short-range order structure defined above, at least some of one or more groups of identical diameter spherical particles (G-PID) are preferably added in the form of an organic-inorganic composite filler including: one group of identical diameter spherical particles (with no other group of identical diameter spherical particles); and a resin having a refractive index smaller than that of the spherical inorganic particles constituting the one group of identical diameter spherical particles (in other words, an organic-inorganic composite filler containing only a single group of identical diameter spherical particles) or preferably added in the form of 5 to 200 µm-diameter aggregates of the spherical inorganic particles.

Meanwhile, Patent Document 5 states that a good opalescent effect can be produced by a product resulting from the curing of a dental restoration composite material including: (A) 100 parts by mass of a polymerizable monomer(s); (B) 100 to 400 parts by mass of spherical silica particles having an average particle diameter in the range of 0.1 to 0.5 µm and having a particle size distribution with a standard deviation of 1.30 or less; (C) 50 to 450 parts by mass of an organic-inorganic composite filler including an organic resin matrix and the silica particles dispersed therein; and (D) 0.01 to 10 parts by mass of a polymerization initiator, in which a product resulting from the curing of the polymerizable monomer (A) and the organic-inorganic composite filler (C) have a refractive index difference of 0.1 or less, and the product resulting from the curing of the polymerizable monomer (A) has a refractive index higher than that of the silica particles.

Patent Documents 4 and 5 describe the details of the respective components of the raw material composition, namely the polymerizable monomer, the inorganic or silica particles, and the polymerization initiator.

Preferred examples of the polymerizable monomer include (meth)acrylic monomers, such as methyl (meth)acrylate, ethyl (meth)acrylate, 2-ethylhexyl (meth)acrylate, (meth)acrylic acid, N-(meth)acryloylglycine, 2-(meth)acryloyloxybenzoic acid, 6-(meth)acryloyloxyethylnaphthalene-1,2,6-tricarboxylic anhydride, 13-(meth)acryloyloxytridecane-1,1-dicarboxylic acid, 2-(meth)acrylamido-2-methylpropanesulfonic acid, 2-hydroxyethyl (meth)acrylate, 3-hydroxypropyl (meth)acrylate, N-hydroxyethyl(meth)acrylamide, N,N-(dihydroxyethyl)(meth)acrylamide, 2,2-bis[methacryloyloxyphenyl]propane, 2,2-bis[(3-methacryloyloxy-2-hydroxypropyloxy)phenyl]propane, ethylene glycol dimethacrylate, triethylene glycol dimethacrylate, 1,6-bis(methacrylethyloxycarbonylamino)trimethylhexane, trimethylolpropane trimethacrylate, pentaerythritol tetramethacrylate, and neopentylglycol dimethacrylate; and vinyl monomers, such as p-vinylbenzoic acid. As used herein, the term "(meth)acrylate" means both "acrylate" and "methacrylate". The same applies to "(meth)acrylic acid", "(meth)acryloyl", "(meth)acrylamide", and other similar terms.

For the production of a cured product with adjusted physical properties, two or more polymerizable monomers are usually used. In such a case, for ease of satisfying the condition n_{(MX)} **<** n_{(G-PIDm)}, the type and amount of the polymerizable monomers are preferably selected so as to form a polymerizable monomer mixture with a refractive index in the range of 1.38 to 1.55. The refractive indices of the polymerizable monomers and the products resulting from the curing of the polymerizable monomers can be determined using an Abbe refractometer.

The spherical inorganic particles for forming G-PID, the inorganic particles for forming G-SFP, and the silica particles may be made of amorphous silica, silica- titanium group element oxide-based composite oxide (e.g., silica-zirconia, silica-titania), or the like. While the silica-titanium group element oxide-based composite oxide may have any composite ratio, it preferably has, for example, a silica content of 70 to 95 mol% and a titanium group element oxide content of 5 to 30 mol%. These inorganic particles may have undergone surface treatment with a silane coupling agent.

Preferred examples of the polymerization initiator include peroxides, such as benzoyl peroxide, p-chlorobenzoyl peroxide, tert-butylperoxy-2-ethyl hexanoate, tert-butylperoxydicarbonate, and diisopropylperoxydicarbonate; azo compounds, such as azobisisobutyronitrile; boron compounds, such as tributylborane, tributylborane partial oxide, sodium tetraphenylborate, sodium tetrakis(p-fluorophenyl)borate, and triethanolamine salts of tetraphenylboronic acid; barbituric acid compounds, such as 5-butylbarbituric acid and 1-benzyl-5-phenylbarbituric acid; sulfinic acid salts, such as sodium benzenesulfinate and sodium p-toluenesulfinate; and other thermal polymerization initiators. These polymerization initiators may be used alone, or a mixture of two or more of these polymerization initiators may be used. Two or more initiators for different types of polymerization may also be used in combination.

The raw material composition may also contain other additives, such as a polymerization inhibitor and an ultraviolet absorber, as long as the desired effect is not impaired.

### Colorant-Containing Structural Color HR and Raw Material Composition Thereof

The HR blank of the present invention has a monolayer- or multilayer-structure workpiece portion having at least one layer including a colorant-containing structural color HR. The colorant-containing structural color HR, which contains a specific amount of a specific colorant, is a product resulting from the curing of a raw material composition (for the colorant-containing structural color HR), which is produced by adding a specific amount of a specific colorant to a (colorant-free) raw material composition (for the structural color HR). Thus, the components other than the specific colorant and the proportions of the components in the raw material composition for the HR blank of the present invention may be as described above in the <Raw Material Composition for Structural Color HR> section with no particular difference to be mentioned.

Hereinafter, therefore, the specific colorant and the specific content thereof will be described. The term "specific colorant" means a colorant including 70 mass% or more of a similarly chromatic coloring material (with a differently chromatic coloring material content of less than 30 mass%) having a coloring wavelength falling within the wavelength region A, which is determined based on the structural color wavelength and the reflectance at the structural color wavelength. The term "specific content" means 800 to 8,000 ppm by mass based on the total amount of the colorant-containing structural color HR. If the colorant includes less than 70 mass% of a similarly chromatic coloring material(s), the HR blank will tend to change its appearance color depending on the thickness of the product resulting from the milling of it. If the content of the colorant is less than 800 ppm by mass, the HR blank will tend to change its appearance color due to the color of the base, which can be seen through it after the milling of it. If the content of the colorant is more than 8,000 ppm by mass, the color derived from the colorant will be so strong before milling as to make it difficult to carry out shade taking based on the appearance color. For the advantageous effect, the content of the colorant is preferably 900 to 6,000 ppm by mass, more preferably 2,000 to 3,500 ppm by mass. The similarly chromatic coloring material(s) may be a mixture of different types of coloring materials. When 1 mm thick, the colorant-containing structural color HR satisfying these conditions has a spectral reflectance curve with a first maximum reflectance ML (%) and a second maximum reflectance MH (%) satisfying the condition: MH/ML (the ratio of MH to ML) > 1.00, preferably the condition: MH/ML > 1.20, as measured on a black background with a colorimeter, wherein the ML is the maximum reflectance in the wavelength range of 400 to 500 nm of the spectral reflectance curve, and the MH is the maximum reflectance in the wavelength range of 600 to 750 nm of the spectral reflectance curve.

The coloring material(s) is preferably a pigment(s) and/or a dye(s). The pigment(s) is typically an inorganic pigment(s), examples of which include titanium dioxide, zinc oxide, zirconium oxide, zinc sulfide, aluminum silicate, calcium silicate, carbon black, iron oxide, copper chromite black, chromium oxide green, chromium green, violet, chromium yellow, lead chromate, lead molybdate, cadmium titanate, nickel titanium yellow, ultramarine blue, cobalt blue, bismuth vanadate, cadmium yellow, and cadmium red. It should be noted that the content of the inorganic pigment, which may correspond to inorganic particles, is much lower than that of G-PID or G-SFP and thus has no influence on that of G-PID or G-SFP. The pigment(s) may also be an organic pigment(s), such as monoazo pigments, diazo pigments, diazo condensation pigments, perylene pigments, and anthraquinone pigments. Examples of the dye include red dyes, such as KAYASET RED G and KAYASET RED B (all manufactured by Nippon Kayaku Co., Ltd.); yellow dyes, such as KAYASET Yellow 2G and KAYASET Yellow GN (all manufactured by Nippon Kayaku Co., Ltd.); and blue dyes, such as KAYASET Blue N, KAYASET Blue G, and KAYASET Blue B (all manufactured by Nippon Kayaku Co., Ltd.). In view of color stability in the oral cavity, the colorant(s) is preferably a pigment(s), which is water-insoluble, rather than a dye(s), which may be water-soluble.

These coloring materials should be classified as the similarly chromatic coloring materials or the differently chromatic coloring materials according to their coloring wavelength before being added to the composition.

The raw material composition for the colorant-containing structural color HR is preferably produced by adding the specific amount of the specific colorant to a raw material composition for a colorant-free, specific structural color HR, so that the resulting product can have an appropriate level of transparency and produce a high structural color effect. In this case, the content of G-PID in the G-SFP-free composition is preferably 100 to 900 parts by mass, more preferably 150 to 700 parts by mass, based on 100 parts by mass of the polymerizable monomer(s). The content of G-PID in the G-SFP-containing composition is preferably the value calculated by subtracting the content of G-SFP from the above G-PID content.

For ease of adjusting the shade to shades A to D, the structural color wavelength is 590 to 690 nm. Thus, the G-PID preferably has an average primary particle diameter of 230 to 800 nm, more preferably 240 to 500 nm, even more preferably 260 to 350 nm. The number a of G-PIDs is preferably 1 to 3, more preferably 1 or 2. In a case where at least some of the G-PIDs is added in the form of an organic-inorganic composite filler, the average particle diameter of the organic-inorganic composite filler is preferably 2 to 100 µm, more preferably 5 to 50 µm, even more preferably 5 to 30 µm, for high mechanical strength of the cured product and for good handleability of the curable paste.

The raw material composition for the colorant-containing structural color HR can be prepared by a process including: kneading specific amounts of the respective components; and defoaming the mixture. In particular, the kneading method is preferably performed using a kneader, such as a planetary stirring machine, so that kneading can be performed to achieve, in a short time, a good dispersion state that makes it possible to produce a cured product satisfying conditions 1 and 2 and so that scale-up production can be easily achieved. The defoaming is preferably performed under reduced pressure, so that air bubbles can be removed in a short time even from a high-viscosity composition.

The resulting raw material composition for the colorant-containing structural color HR is polymerized and cured by action of the polymerization initiator to produce the colorant-containing structural color HR. In a case where a thermal polymerization initiator is used, the polymerization temperature is preferably 70 to 150°C, more preferably 80 to 130°C, so that the polymerization can be allowed to proceed sufficiently without thermal discoloration and that the colorant-containing structural color HR can be obtained with high strength.

### Method for Producing the HR blank of the Present Invention

The HR blank of the present invention is advantageously produced by cast-molding polymerization of the raw material composition for the colorant-containing structural color HR, specifically, for the colorant-containing, specific structural color HR, no matter whether the workpiece portion has a monolayer structure or a multilayer structure.

Specifically, the HR blank of the present invention is preferably produced by a method including the steps (i) to (iii):
(i) mixing 100 parts by mass of a polymerizable monomer(s); 10 to 1,500 parts by mass of one or more groups of identical diameter spherical particles (G-PID); and 0.01 to 10 parts by mass of a polymerization initiator, and optionally 0.1 to 50 parts by mass of a group of superfine particles (G-SFP) by the method (a) or (b) described above to form a raw material composition for colorant-free, specific structural color HR;
(ii) adding, to the raw material composition, 800 to 8,000 ppm by mass of a colorant including 70 mass% or more of a similarly chromatic coloring material based on the total mass of the raw material composition and the colorant to form a raw material composition for colorant-containing, specific structural color HR, wherein the similarly chromatic coloring material is defined by the following manner: in a spectral reflectance curve of the colorant-free structural color HR obtained by measuring a 3 mm-thick sample of the colorant-free structural color HR on a black background with a colorimeter, in which a horizontal axis represents reflected light wavelength (nm) and a vertical axis represents reflectance (%), a wavelength at which a maximum reflectance derived from the structural color occurs is defined as a structural color wavelength and a wavelength region in which 85% or more of the maximum reflectance occurs is defined as a wavelength region A; and in spectral reflectance curve of a coloring material, of the one or more coloring materials, obtained by measurement on a black background with a colorimeter, a wavelength at which a maximum reflectance occurs is defined as a coloring wavelength of the coloring material, a coloring material, of the one or more coloring materials, whose coloring wavelength exists in the wavelength region A is defined as a similarly chromatic coloring material, and a coloring material, of the one or more coloring materials, whose coloring wavelength exists outside the wavelength region A is defined as a differently chromatic coloring material; and
(iii) subjecting the raw material composition for colorant-containing, specific structural color HR to cast molding polymerization.

As used herein, the term "cast molding polymerization" means a process including: charging the polymerizable curable composition into a mold with a specific shape; and then polymerizing and curing the composition. The mold may have any suitable volume depending on the desired shape. The mold may also have any suitable shape, such as a square cylinder, a cylinder, a square plate, a disk, or any other irregular shape. During the polymerization, if necessary, the composition may be pressurized with inert gas, such as nitrogen. The mold provided may have the same shape or substantially the same shape as the workpiece portion to be formed. The curable raw material compositions for forming layers may be sequentially charged with predetermined thicknesses into the interior of the mold and then polymerized and cured to form a bulk body (a stack of HRs), which may be used as the workpiece portion without any modification. Alternatively, the curable raw material compositions may be charged into a mold with a size larger than that of the above and then polymerized and cured to form a bulk body, which may be punched or milled to form the workpiece portion. The composition may be charged into the mold by any known technique or method, such as injection, extrusion, or pressing.

The mold may be made of metal, ceramic, or resin depending on the purpose, and is preferably made of a heat-resistant material capable of withstanding temperatures higher than the practical polymerization temperature. For example, the mold may be made of SUS, high-speed tool steel, aluminum alloy, polyethylene terephthalate (PET), polyethylene (PE), polypropylene (PP), or polystyrene (PS).

The resulting bulk body may be subjected to post-processes, such as heat treatment, polishing, milling, attachment of fixing members, and printing, as needed. Moreover, if necessary, a fixing pin may be bonded to the blank so that the resulting blank for dental milling can be fixed through it to a milling machine. The fixing pin may have any shape suitable for fixing, to a milling machine, the resulting blank for dental milling. There may be no need for any fixing pin depending on the requirements of the milling machine and the shape of the blank for dental milling. The fixing pin may be made of stainless steel, brass, or aluminum. The fixing pin may be bonded to the workpiece portion (the main body of the blank for dental milling) not only by adhesive bonding but also by fitting, screwing, or any other method. The adhesive bonding may also be any type using any commercially available adhesive, such as an isocyanate, epoxy, urethane, silicone, or acrylic adhesive.

### Method for Fabricating Crown

The HR blank of the present invention is less likely to change its appearance color tone before and after being milled into a small thickness. Thus, the HR blank of the present invention is suitable for use in fabricating crowns.

The crown may be fabricated by any suitable method. For example, the crown for use on an anchor tooth is preferably formed by milling the workpiece portion of the HR blank of the present invention by CAD/CAM technique using three-dimensional, intraoral shape data on the patient having the anchor tooth.

After the milling, the crown may be further modified in shape and polished on surface using a mechanic engine or the like. If necessary, the color tone of the crown may be adjusted using a penetrating colorant, a clarifying solution, or the like.

### EXAMPLES

The present invention will be more specifically described with reference to examples, which are not intended to limit the present invention.

### 1. Raw Materials for Hybrid Resin

The raw materials for the hybrid resins used in the examples and comparative examples and their physical properties will be described below.

### (1) Polymerizable Monomer Component

A mixture M1 of polymerizable monomers in the proportion shown in Table 1 was used as a polymerizable monomer component. In this regard, the abbreviations in the polymerizable monomer column of the table represent the following compounds, respectively, and the numbers in the parentheses indicate their amounts (parts by mass).
UDMA: 1,6-Bis(methacrylethyloxycarbonylamino)trimethylhexane
3G: Triethylene glycol dimethacrylate

The refractive indices of the uncured material (M1) and a cured product (cured sample) were measured in a thermostatic chamber at 25°C using an Abbe refractometer (manufactured by ATAGO MFG. Co., Ltd.). The cured sample was prepared by a process including: uniformly mixing 100 parts by mass of the polymerizable monomer mixture M1, 0.2 mass% of camphorquinone (CQ), 0.3 mass% of ethyl p-N,N-dimethylaminobenzoate (DMBE), and 0.15 mass% of hydroquinone monomethyl ether (HQME); adding the mixture to a mold having a through hole of 7 mmφ × 0.5 mm; bringing polyester films into press-contact with both surfaces of the mixture; then curing the mixture by photoirradiation at a dose of 500 mW/cm² for 30 seconds using a halogen dental irradiator (Demetron LC manufactured by Sybron); and removing the cured product from the mold. When the cured sample was placed in the Abbe refractometer, a solvent (bromonaphthalene) having a refractive index higher than that of the sample and not being capable of dissolving the sample was dropped on the sample to allow the sample to adhere to the measurement surface.

**[Table 1]**

| | Polymerizable monomer | Refractive index | |
|---|---|---|---|
| | | Before curing | After curing |
| M1 | UDMA(80)/3G(20) | 1.479 | 1.509 |

### (2) Group of Identical Diameter Spherical Particles (G-PID)

According to the method disclosed in the "Examples" section of Patent Document 4, a spherical filler of a composite oxide composed of SiO₂, ZrO₂, and Na₂O in a molar ratio of 89.8:9.0:1.2 was prepared by sol-gel method and then surface-treated with γ-methacryloyloxypropyltrimethoxysilane to form each of G-PID1 to G-PID6 shown in Table 2.

### (3) Irregular Shaped Inorganic Filler

The irregular shaped inorganic filler F1 (irregular shaped silica-zirconia particles) shown in Table 2 was prepared by the method disclosed in Japanese Unexamined Patent Application, Publication No. H2-132102 or H3-197311 including: dissolving an alkoxysilane compound in an organic solvent; adding water to the solution to allow the alkoxysilane compound to undergo partial hydrolysis; adding, to the product, another metal alkoxide and an alkali-metal compound for forming a composite; hydrolyzing the mixture to form a gel-like material; then dying the gel-like material and optionally griding the dried material; and firing the material.

**[Table 2]**

| | Refractive index | Average primary particle diameter (µm) | Average uniformity | ±5% Range particle percentage (%) |
|---|---|---|---|---|
| G-PID1 | 1. 52 | 0.30 | 0.91 | 91 |
| G-PID2 | 1. 52 | 0.28 | 0.92 | 90 |
| G-PID3 | 1. 52 | 0.25 | 0.88 | 95 |
| G-PID4 | 1. 52 | 0.20 | 0.90 | 92 |
| G-PID5 | 1. 54 | 0.15 | 0.90 | 91 |
| G-PID6 | 1. 52 | 0.32 | 0.91 | 91 |
| F1 | 1. 52 | 0.50 | - | 50 |

Table 2 shows the average primary particle diameter, the average uniformity, the ±5% range particle percentage (the ratio (%) of the number of particles in the range of ±5% of the average primary particle diameter to the total number of particles in the number-based particle size distribution), and the refractive index, which were measured by the methods shown in the "Examples" section of Patent Document 4.

Specifically, the average primary particle diameter means the value determined by a process including: subjecting, to image processing, a powder photograph taken with a scanning electron microscope (magnification: 5,000 to 100,000×); measuring the diameters (maximum diameters) of 30 or more primary particles observed in a unit field of view of the resulting image; and dividing the sum of the measured diameters by the number of the particles.

The average uniformity means the value determined by a process including: measuring the long diameters (Li) of the 30 or more particles; measuring their short diameters (Bi) perpendicular to the long diameters; and dividing the sum of the short-diameter (Bi) to long-diameter (Li) ratios (Bi/Li) by the number of the particles.

The ±5% range particle percentage means the value calculated according to the formula below. The ±5% range particle percentage (%) = (N₁/N₀) × 100
N₁: The number of particles with diameters in the range of ±5% of the average primary particle diameter in a unit field of view of a scanning electron micrograph.
N₀: The total number of particles in the unit field of view of the scanning electron micrograph.

The refractive index means the value measured at 25°C with sodium D line by an immersion method including measuring the refractive index of a dispersion prepared by dispersing particles in anhydrous toluene (dispersion medium) and clarifying the dispersion with a drop of 1-bromotoluene.

### (3) Organic-Inorganic Composite Filler

The organic-inorganic composite fillers CF1 and CF2 were prepared as shown below using G-PID2 and G-PID5 shown in Table 2, respectively. First, using a circulation-type grinder SC mill (manufactured by Nippon Coke & Engineering. Co., Ltd.), 100 g of G-PID2 or G-PID5 was dispersed in 200 g of water to form a dispersion. Next, a solution (pH 4) of a mixture of 4 g of γ-methacryloyloxypropyltrimethoxysilane, 0.003 g of acetic acid, and 80 g of water, which was separately prepared, was added to the dispersion, and mixed until uniform. The resulting dispersion being mixed lightly was supplied onto a disk rotating at high speed and subjected to granulation by spray drying. The resulting powder was vacuum-dried at 60°C to give substantially spherical aggregates. Next, 50 g of the aggregates were impregnated with a polymerizable monomer solution prepared by mixing 10 g of the polymerizable monomer mixture M1, 0.025 g of azobisisobutyronitrile (AIBN) (thermal polymerization initiator), and 5.0 g of methanol (organic solvent). After sufficient stirring, the organic solvent was removed from the impregnated product. The resulting product was heated under conditions at a reduced pressure of 10 hPa and 100°C for 1 hour so that the polymerizable monomer mixture was polymerized and cured. This process produced the organic-inorganic composite filler CF1 or CF2 including substantially spherical particles with an average diameter of 10 µm.

### (4) Superfine Particles (G-SFP)

The G-SFP was Reolosil QS-102 (12 nm in average primary particle diameter, manufactured by Tokuyama Corporation).

### (5) Polymerization Initiator

The thermal polymerization initiator was benzoyl peroxide (BPO).

### (6) Colorant

Pigments with three colors R (red), Y (yellow), and B (blue) shown below were mixed in the mass ratios shown in Table 3 to form colorants G1, G2, G3, G4, G5, and G6, which were put into use. The coloring wavelength of each pigment was determined as the wavelength at which the maximum reflectance occurred in the spectral reflectance curve obtained by measuring, on a black background, the spectral reflectance of a 3 mm-thick HR blank sample resulting from the curing of the composition containing 2 to 10 mass% of the pigment alone (exhibiting no structural color) based on the total mass of the composition.
R: Red pigment (Pigment Red 166) with a coloring wavelength of 680 nm
Y: Yellow pigment (Pigment Yellow 95) with a coloring wavelength of 590 nm
B: Blue pigment (Pigment Blue 60) with a coloring wavelength of 430 nm

Table 3 shows the proportion of each pigment in units of ppm by mass, which means the content (ppm by mass) of the pigment in the colorant-containing structural color HR, prepared in the examples and the comparative examples shown later, based on the total mass of the HR. The term "reference shade" in Table 3 means the shade determined using a VITA shade guide for the HR blank of each of the examples and the comparative examples shown later (each containing only one of G1 to G6 according to the above). In other words, the composition (in units of ppm by mass) of each of G1 to G6 was determined such that the corresponding reference shade was achieved using only one of G1 to G6 in the preparation of the HR blank of each of the examples and the comparative examples shown below.

**[Table 3]**

| | Colorant (ppm by mass) | | | | Reference shade |
|---|---|---|---|---|---|
| | Y | R | B | Total | |
| G1 | 600 | 120 | 80 | 800 | B1 |
| G2 | 650 | 150 | 100 | 900 | A1 |
| G3 | 1050 | 350 | 550 | 1950 | A2 |
| G4 | 1800 | 650 | 1000 | 3450 | A3 |
| G5 | 3000 | 1000 | 1600 | 5600 | A4 |
| G6 | 3700 | 1900 | 2400 | 8000 | C4 |

### 2. Examples and Comparative Examples

### <Example 1>

### (1) Preparation of Colorant-Containing Structural Color HR

A paste was prepared by mixing 20 parts by mass of the polymerizable monomer mixture M1 and 1.0 part by mass of BPO, then adding, to the mixture, 80 parts by mass of G-PID1, 1 part by mass of G-SFP, and the colorant G4 in the amount (ppm by mass) shown in Table 3, and mixing the materials until uniform using a planetary mixer. The resulting paste was defoamed in vacuum and then charged into the substantially rectangular columnar cavity of a mold having a thickness of 14.5 mm and a cross section of 14.5 mm × 18 mm. The paste was pressurized with nitrogen at 0.4 MPa in a pressurized vessel and allowed to stand at 90°C in a heating apparatus. In that state, the paste was heated for 15 hours to undergo polymerization and curing. The product was cooled to room temperature at a cooling rate of 20°C/min and then taken out of the mold to give a block-shaped, colorant-containing, structural color HR (cured product) with a shape corresponding to the shape of the cavity.

### (2) Measurement of Structural Color Wavelength and Determination of Similarly Chromatic Pigment Content and Differently Chromatic Pigment Content

A colorant-free structural color HR (hereinafter also referred to as "base structural color HR") was obtained as a block-shaped cured product as in (1) preparation of the colorant-containing structural color HR except that the colorant G4 was not added. The resulting block-shaped cured product was cut into a rectangular plate-shaped piece with a thickness of 3 mm and a main surface of 12 mm × 14 mm, which was polished to gloss to give a solid sample for structural color wavelength measurement. The spectral reflectance of the solid sample for structural color wavelength measurement was measured on a black background formed of black carbon tapes using a spectrophotometer (TC-1800MKII manufactured by Tokyo Denshoku Co., Ltd.; halogen lamp: 12 V, 100 W; measurement wavelength range: 380 to 780 nm) to give a spectral reflectance curve. The structural color wavelength of the sample was determined to be 670 nm, which was the wavelength at which the maximum reflectance occurred in the resulting spectral reflectance curve. The wavelength region A in which 85% or more of the maximum reflectance occurred in the spectral reflectance curve was determined to be the range of 590 to 750 nm. These results indicate that Y and R in the colorant-containing structural color HR of Example 1 are similarly chromatic pigments, B is a differently chromatic pigment, and the content of the similarly chromatic pigments in the colorant is 71 mass%.

### (3) Determination of MH/ML

The block-shaped, colorant-containing structural color HR (cured product) resulting from the preparation (1) was cut into a rectangular plate-shaped piece with a thickness of 1 mm and a main surface of 12 mm × 14 mm, which was polished to gloss to give a solid sample (1 mm in thickness). The solid sample was measured on a black background with a colorimeter. In the resulting spectral reflectance curve, the maximum reflectance ML (%) in the wavelength range of 400 to 500 nm and the maximum reflectance MH (%) in the wavelength range of 600 to 750 nm were determined, and the ratio (MH/ML) of MH to ML was calculated to be 1.13.

### (4) Evaluation of Color Tone Compatibility of Colorant-Containing Structural Color HR

The block-shaped, colorant-containing, structural color HR (cured product) was named sample 1, which was subjected to appearance color comparison as shown below. Subsequently, the colorant-containing structural color HR was cut into a rectangular plate-shaped piece with a thickness of 2 mm and a main surface of 12 mm × 14 mm, which was polished to gloss to give a solid sample (sample 2). The appearance color was compared between samples 1 and 2 as shown below. The appearance comparison was made by aligning each of the samples and VITA shade guide A3 (corresponding to the colorant G4) on a black background and performing visual evaluation to compare their appearance colors. Based on the comparison, the color tone compatibility was evaluated according to the criteria shown below. As a result, sample 1 was rated "A", while sample 2 was rated "B".

### -Evaluation Criteria-

A: The color tone of the sample is highly identical with that of the VITA shade guide.
B: The color tone of the sample is similar to that of the VITA shade guide.
C: The color tone of the sample is similar to that of the VITA shade guide, but not well compatible with that of the VITA shade guide.
D: The color tone of the sample is not compatible with that of the VITA shade guide.

### <Examples 2 to 19 and Comparative Examples 1 to 13>

In each of Examples 2 to 19 and Comparative Examples 1 to 13, a paste (raw material composition for base structural color HR) was prepared as in Example 1 except that G-PID1 (80 parts by mass) for Example 1 was replaced with the type and amount (in units of parts by mass based on 20 parts by mass of the polymerizable monomer mixture M1) of inorganic particles (or a combination of inorganic particles and an organic-inorganic composite filler) shown in the "Inorganic particles" column of each of Tables 4 and 5. As indicated by the footnotes of Tables 4 and 5, the number in the parentheses indicates the amount of the organic-inorganic filler (in units of parts by mass). The content (parts by mass) of inorganic particles in the organic-inorganic composite filler is 80% of the parenthesized number. The resulting paste (raw material composition for base structural color HR) was used to form a sample, and the structural color wavelength and wavelength region A of the sample were measured according to the section (2) of Example 1. Tables 4 and 5 also shown the results.

In each example, a block-shaped, colorant-containing, structural color HR (cured product) was prepared as in Example 1 except that the type and amount of the colorant shown in each of Tables 6 and 7 was added instead to the resulting raw material composition for base structural color HR, and the resulting cured product was measured and evaluated as in Example 1. The results are shown in Tables 6 and 7. In tables 6 and 7, the column titled "before milling" shows the results of color tone compatibility evaluation of sample 1, while the column titled "after milling" shows the results of color tone compatibility evaluation of sample 2. The VITA shade guide for appearance color comparison was selected to correspond to the colorant reference shade (shown in Table 3) depending on the colorant (G1 to G6) used.

**[Table 4]**

| | | Inorganic particles (parts by mass)* | Structural color wavelength (nm) | Wavelength region A (nm) |
|---|---|---|---|---|
| | 1 | G-PID1 (80) | 670 | 590-750 |
| | 2 | G-PID2 (80) | 650 | 550-700 |
| | 3 | G-PID3 (80) | 590 | 510-680 |
| | 4 | G-PID1 (40) G-PID3 (40) | 659 | 570-740 |
| | 5 | G-PID2 (64) G-PID3 (16) | 645 | 540-690 |
| | 6 | G-PID2 (40) G-PID3 (40) | 630 | 530-685 |
| | 7 | G-PID2 (64) G-PID5 (16) | 640 | 535-690 |
| | 8 | G-PID2 (64) CF1 (16) | 660 | 530-700 |
| | 9 | G-PID2 (40) CF1 (40) | 655 | 500-700 |
| Example | 10 | G-PID2 (80) | 650 | 550-700 |
| | 11 | G-PID2 (80) | 650 | 550-700 |
| | 12 | G-PID2 (80) | 650 | 550-700 |
| | 13 | G-PID2 (80) | 650 | 550-700 |
| | 14 | G-PID2 (80) | 650 | 550-700 |
| | 15 | G-PID2 (40) CF1 (40) | 655 | 500-700 |
| | 16 | G-PID2 (40) CF1 (40) | 655 | 500-700 |
| | 17 | G-PID2 (40) CF1 (40) | 655 | 500-700 |
| | 18 | G-PID2 (40) CF1 (40) | 655 | 500-700 |
| | 19 | G-PID2 (40) CF1 (40) | 655 | 500-700 |

| | | | | |
|---|---|---|---|---|
| * CF1 is shown with the total mass of the organic-inorganic composite filler. | | | | |

**[Table 5]**

| | | Inorganic particles (parts by mass)* | Structural color wavelength (nm) | Wavelength region A (nm) |
|---|---|---|---|---|
| | 1 | G-PID4 (80) | 510 | 480-590 |
| | 2 | G-PID5 (80) | 430 | 410-450 |
| | 3 | G-PID6 (80) | 720 | 630-770 |
| | 4 | G-PID5 (40) CF2 (40) | 440 | 410-480 |
| | 5 | G-PID5 (80) | 430 | 410-450 |
| Comparative Example | 6 | G-PID5 (80) | 430 | 410-450 |
| | 7 | G-PID5 (80) | 430 | 410-450 |
| | 8 | G-PID5 (80) | 430 | 410-450 |
| | 9 | G-PID5 (80) | 430 | 410-450 |
| | 10 | F1 (80) | - | - |
| | 11 | F1 (80) | - | - |
| | 12 | F1 (80) | - | - |
| | 13 | F1 (80) | - | - |

| | | | | |
|---|---|---|---|---|
| * CF2 is shown with the total mass of the organic-inorganic composite filler. | | | | |

**[Table 6]**

| | | Structural color wavelength of base structural color HR (nm) | Colorant (ppm by mass) * | Content in colorant (mass%) | | MH/ML | Color tone compatibility evaluation | |
|---|---|---|---|---|---|---|---|---|
| | | | | Similarly chromatic | Differently chromatic | | Before milling | After milling |
| | 1 | 670 | G4 (3450) | 71 | 29 | 1.13 | A | B |
| | 2 | 650 | G4 (3450) | 71 | 29 | 1.15 | A | A |
| | 3 | 590 | G4 (3450) | 71 | 29 | 1.09 | A | B |
| | 4 | 659 | G4 (3450) | 71 | 29 | 1.08 | B | B |
| | 5 | 645 | G4 (3450) | 71 | 29 | 1.05 | A | B |
| | 6 | 630 | G4 (3450) | 71 | 29 | 1.09 | A | B |
| | 7 | 640 | G4 (3450) | 71 | 29 | 1.01 | B | B |
| | 8 | 660 | G4 (3450) | 71 | 29 | 1.14 | A | A |
| | 9 | 655 | G4 (3450) | 71 | 29 | 1.14 | A | A |
| Example | 10 | 650 | G1 (800) | 90 | 10 | 1.08 | B | B |
| | 11 | 650 | G2 (500) | 89 | 11 | 1.05 | A | B |
| | 12 | 650 | G3 (1950) | 72 | 28 | 1.07 | A | A |
| | 13 | 650 | G5 (5600) | 71 | 29 | 1.25 | A | A |
| | 14 | 650 | G6 (8000) | 70 | 30 | 1.09 | B | B |
| | 15 | 655 | G1 (800) | 90 | 10 | 1.08 | B | B |
| | 16 | 655 | G2 (900) | 89 | 11 | 1.03 | A | B |
| | 17 | 655 | G3 (1950) | 72 | 28 | 1.10 | A | A |
| | 18 | 655 | G5 (5600) | 71 | 29 | 1.23 | A | A |
| | 19 | 655 | G6 (8000) | 70 | 30 | 1.07 | B | B |

| | | | | | | | | |
|---|---|---|---|---|---|---|---|---|
| * The colorant content is in units of ppm by mass based on the total mass of the composition. | | | | | | | | |

| | | Structural color wavelength of base structural color HR (nm) | Colorant (ppm by mass) * | Content in colorant (mass%) | | MH/ML | Color tone compatibility evaluation | |
|---|---|---|---|---|---|---|---|---|
| | | | | Similarly chromatic | Differently chromatic | | Before milling | After milling |
| | 1 | 510 | G4 (3450) | 48 | 52 | 0.95 | B | C |
| | 2 | 430 | G4 (3450) | 29 | 71 | 0.86 | B | D |
| | 3 | 720 | G4 (3450) | 19 | 81 | 1.05 | B | C |
| | 4 | 440 | G4 (3450) | 29 | 71 | 0.85 | B | D |
| | 5 | 430 | G1 (800) | 10 | 90 | 0.52 | B | D |
| Comparative Example | 6 | 430 | G2 (900) | 11 | 89 | 0.45 | B | D |
| | 7 | 430 | G3 (1950) | 28 | 72 | 0.78 | B | D |
| | 8 | 430 | G5 (5600) | 29 | 71 | 0.84 | B | D |
| | 9 | 430 | G6 (8000) | 30 | 70 | 0.82 | B | D |
| | 10 | 430 | G2 (900) | 89 | 11 | - | A | D |
| | 11 | 430 | G3 (1950) | 72 | 28 | - | A | C |
| | 12 | 430 | G4 (3450) | 48 | 52 | - | B | C |
| | 13 | 430 | G5 (5600) | 71 | 29 | - | B | D |

| | | | | | | | | |
|---|---|---|---|---|---|---|---|---|
| * The colorant content is in units of ppm by mass based on the total mass of the composition. | | | | | | | | |

Tables 6 and 7 indicate that the samples of Examples 1 to 19, prepared using an inorganic particle composition having a structural color wavelength of 590 to 690 nm, each exhibit good color tone compatibility before and after milling. In all of Examples 1 to 19, the content of similarly chromatic pigments in the colorant is at least 70 mass%, and the MH/ML value is at least 1.00.

In contrast, the samples of Comparative Examples 1 to 9 were all rated "C" or "D" after milling in the evaluation of color tone compatibility because the inorganic particle composition used had a structural color wavelength of less than 590 nm or more than 690 nm and the content of differently chromatic pigments in the colorant was as high as more than 30 mass%. In particular, the samples of Comparative Examples 2 and 4 to 9 were rated "D" after milling in the evaluation of color tone compatibility because the samples exhibited a blueish structural color with a relatively short wavelength, the resulting content of similarly chromatic pigments in the colorant was less than 30 mass% (the differently chromatic pigment content was more than 70 mass%), and the MH/ML value was much less than 1.

The samples of Comparative Examples 10 to 13, prepared with a HR blank containing the irregular shaped filler F1 (inorganic particles) (a common blank exhibiting no structural color), significantly changed their appearance color depending on the change in sample thickness and showed a large difference before and after milling in the evaluated color tone compatibility. For example, the color tone compatibility evaluation rate of the sample of Comparative Example 10 was "A" before milling but worsened to "D" after milling.

## Claims

1. A hybrid resin blank for dental milling, the hybrid resin blank comprising: a monolayer- or multilayer-structure workpiece portion comprising a layer of a colorant-containing structural color hybrid resin comprising: a resin matrix of a structural color hybrid resin containing dispersed inorganic particles and exhibiting a structural color with a specific color tone; and a colorant including one or more coloring materials and dispersed in the resin matrix,
the colorant-containing structural color hybrid resin containing 800 to 8,000 ppm by mass of the colorant based on the total mass of the colorant-containing structural color hybrid resin, the colorant comprising 70 mass% or more of a similarly chromatic coloring material, as determined by the following manner:
in a spectral reflectance curve of the colorant-free structural color hybrid resin obtained by measuring a 3 mm-thick sample of the colorant-free structural color hybrid resin on a black background with a colorimeter, in which a horizontal axis represents reflected light wavelength (nm) and a vertical axis represents reflectance in percent, a wavelength at which a maximum reflectance derived from the structural color occurs is defined as a structural color wavelength and a wavelength region in which 85% or more of the maximum reflectance occurs is defined as a wavelength region A; and
in spectral reflectance curve of a coloring material, of the one or more coloring materials, obtained by measurement on a black background with a colorimeter, a wavelength at which a maximum reflectance occurs is defined as a coloring wavelength of the coloring material, a coloring material, of the one or more coloring materials, whose coloring wavelength exists in the wavelength region A is defined as a similarly chromatic coloring material, and a coloring material, of the one or more coloring materials, whose coloring wavelength exists outside the wavelength region A is defined as a differently chromatic coloring material,
wherein the hybrid resin blank has a structural color wavelength in a range of 590 to 690 nm;
**characterised in that**, when 1 mm thick, the colorant-containing structural color hybrid resin has a spectral reflectance curve with a first maximum reflectance ML in percent and a second maximum reflectance MH in percent satisfying the condition:
MH/ML > 1.00 as measured on a black background with a colorimeter, wherein the ML is a maximum reflectance in a wavelength range of 400 to 500 nm of the spectral reflectance curve, and the MH is a maximum reflectance in a wavelength range of 600 to 750 nm of the spectral reflectance curve.

2. The hybrid resin blank according to claim 1, wherein the workpiece portion has a monolayer structure comprising the colorant-containing structural color hybrid resin.

3. The hybrid resin blank according to claim 1, wherein the workpiece portion has a multilayer structure comprising two or three layers including: a first layer that is provided at one end of the multilayer structure and comprises the colorant-containing structural color hybrid resin; and a second layer that comprises another colorant-containing structural color hybrid resin having an appearance color different from that of the colorant-containing structural color hybrid resin, a colorant-free structural color hybrid resin, or a hybrid resin other than these hybrid resins.

4. A method for fabricating a crown, the method comprising: milling the workpiece portion of the hybrid resin blank according to claim 1 by a CAD/CAM technique based on intraoral three-dimensional shape data on a patient having a shaped anchor tooth to fabricate a crown to be attached to the anchor tooth.

## Patentansprüche

1. Hybridkunststoff-Rohling zum dentalen Fräsen, wobei der Hybridkunststoff-Rohling umfasst: einen Werkstückabschnitt mit einlagiger oder mehrlagiger Struktur, umfassend eine Schicht aus einem farbstoffhaltigen Strukturfarben-Hybridkunststoff, der umfasst: eine Harzmatrix eines Strukturfarben-Hybridkunststoffs, die dispergierte anorganische Partikel enthält und eine Strukturfarbe mit einem spezifischen Farbton aufweist; und einen Farbstoff, der ein oder mehrere Farbmittel umfasst und in der Harzmatrix dispergiert ist,
wobei der farbstoffhaltige Strukturfarben-Hybridkunststoff 800 bis 8.000 ppm pro Masse des Farbstoffs, bezogen auf die Gesamtmasse des farbstoffhaltigen Strukturfarben-Hybridkunststoffs, enthält, wobei der Farbstoff zu 70 Masse-% oder mehr ein gleichchromatisches Farbmittel aufweist, bestimmt auf die folgende Weise:
in einer spektralen Reflexionskurve des farbstofffreien Strukturfarben-Hybridkunststoffs, die durch Messung einer 3 mm dicken Probe des farbstofffreien Strukturfarben-Hybridkunststoffs auf schwarzem Hintergrund mittels eines Kolorimeters erhalten wird, wobei die horizontale Achse die Wellenlänge (nm) des reflektierten Lichts und die vertikale Achse die Reflexion in Prozent darstellt, wird eine Wellenlänge, bei der ein von der Strukturfarbe herrührendes Reflexionsmaximum auftritt, als Strukturfarben-Wellenlänge definiert, und ein Wellenlängenbereich, in dem 85 % oder mehr des Reflexionsmaximums auftreten, wird als Wellenlängenbereich A definiert; und
in einer spektralen Reflexionskurve eines Farbmittels des einen oder der mehreren Farbmittel, die durch Messung auf schwarzem Hintergrund mittels eines Kolorimeters erhalten wird, wird eine Wellenlänge, bei der ein Reflexionsmaximum auftritt, als Farbmittel-Wellenlänge des Farbmittels definiert, ein Farbmittel des einen oder der mehreren Farbmittel, dessen Farbmittel-Wellenlänge innerhalb des Wellenlängenbereichs A liegt, wird als gleichchromatisches Farbmittel definiert, und ein Farbmittel des einen oder der mehreren Farbmittel, dessen Farbmittel-Wellenlänge außerhalb des Wellenlängenbereichs A liegt, wird als verschiedenchromatisches Farbmittel definiert,
wobei der Hybridkunststoff-Rohling eine Strukturfarben-Wellenlänge im Bereich von 590 bis 690 nm aufweist;
**dadurch gekennzeichnet, dass** der farbstoffhaltige Strukturfarben-Hybridkunststoff bei einer Dicke von 1 mm eine spektrale Reflexionskurve mit einem ersten Reflexionsmaximum ML in Prozent und einem zweiten Reflexionsmaximum MH in Prozent aufweist, die folgende Bedingung erfüllen: MH/ML > 1,00, gemessen auf schwarzem Hintergrund mittels eines Kolorimeters, wobei ML das Reflexionsmaximum im Wellenlängenbereich von 400 bis 500 nm der spektralen Reflexionskurve ist und MH das Reflexionsmaximum im Wellenlängenbereich von 600 bis 750 nm der spektralen Reflexionskurve ist.

2. Hybridkunststoff-Rohling nach Anspruch 1, wobei der Werkstückabschnitt eine einlagige Struktur umfasst, die den farbstoffhaltigen Strukturfarben-Hybridkunststoff umfasst.

3. Hybridkunststoff-Rohling nach Anspruch 1, wobei der Werkstückabschnitt eine mehrlagige Struktur mit zwei oder drei Schichten umfasst, aufweisend: eine erste Schicht, die an einem Ende der mehrlagigen Struktur vorgesehen ist und den farbstoffhaltigen Strukturfarben-Hybridkunststoff umfasst; und eine zweite Schicht, die einen weiteren farbstoffhaltigen Strukturfarben-Hybridkunststoff mit einer von derjenigen des farbstoffhaltigen Strukturfarben-Hybridkunststoffs verschiedenen Erscheinungsfarbe, einen farbstofffreien Strukturfarben-Hybridkunststoff oder einen von diesen Hybridkunststoffen verschiedenen Hybridkunststoff umfasst.

4. Verfahren zur Herstellung einer Krone, wobei das Verfahren umfasst: Fräsen des Werkstückabschnitts des Hybridkunststoff-Rohlings nach Anspruch 1 mittels einer CAD/CAM-Technik auf Grundlage intraoraler dreidimensionaler Formdaten eines Patienten mit einem präparierten Pfeilerzahn, um eine an dem Pfeilerzahn zu befestigende Krone herzustellen.

## Revendications

1. Ébauche en matière plastique hybride pour fraisage dentaire, l'ébauche en matière plastique hybride comprenant : une section de pièce d'usinage à structure monocouche ou multicouche, comprenant une couche d'une matière plastique hybride à couleur structurelle contenant un colorant, comprenant : une matrice de résine d'une matière plastique hybride à couleur structurelle contenant des particules inorganiques dispersées et présentant une couleur structurelle avec une teinte spécifique ; et un colorant comprenant un ou plusieurs matériaux colorants et dispersé dans la matrice de résine,
la matière plastique hybride à couleur structurelle contenant un colorant, par rapport à la masse totale de la matière plastique hybride à couleur structurelle contenant un colorant, contenant de 800 à 8 000 ppm en masse du colorant, le colorant comprenant 70 % par masse ou plus d'un matériau colorant à chromaticité similaire, déterminé de la manière suivante :
dans une courbe de réflectance spectrale de la matière plastique hybride à couleur structurelle exempte de colorant, obtenue par mesure d'un échantillon de 3 mm d'épaisseur de ladite matière plastique hybride à couleur structurelle exempte de colorant sur fond noir à l'aide d'un colorimètre, dans laquelle l'axe horizontal représente la longueur d'onde (nm) de la lumière réfléchie et l'axe vertical le pourcentage de réflectance, la longueur d'onde à laquelle apparaît un maximum de réflectance dû à la couleur structurelle est définie comme longueur d'onde à couleur structurelle, et une plage de longueurs d'onde dans laquelle 85 % ou plus du maximum de réflectance apparaît est définie comme plage de longueurs d'onde A ; et
dans une courbe de réflectance spectrale d'un matériau colorant parmi le ou les matériaux colorants, obtenue par mesure sur fond noir à l'aide d'un colorimètre, la longueur d'onde à laquelle apparaît un maximum de réflectance est définie comme longueur d'onde de matériau colorant du matériau colorant, un matériau colorant parmi le ou les matériaux colorants dont la longueur d'onde de matériau colorant se situe dans la plage de longueurs d'onde A est défini comme matériau colorant à chromaticité similaire, et un matériau colorant parmi le ou les matériaux colorants dont la longueur d'onde de matériau colorant se situe en dehors de la plage de longueurs d'onde A est défini comme matériau colorant à chromaticité différente,
dans laquelle l'ébauche en matière plastique hybride présente une longueur d'onde à couleur structurelle comprise entre 590 et 690 nm ;
**caractérisé en ce que**, la matière plastique hybride à couleur structurelle contenant un colorant, d'une épaisseur de 1 mm, présente une courbe de réflectance spectrale avec un premier maximum de réflectance ML en % et un second maximum de réflectance MH en %, satisfaisant la condition : MH/ML > 1,00, mesurée sur fond noir à l'aide d'un colorimètre, ML étant le maximum de réflectance dans la plage de longueurs d'onde de 400 à 500 nm de la courbe de réflectance spectrale, et MH étant le maximum de réflectance dans la plage de longueurs d'onde de 600 à 750 nm de la courbe de réflectance spectrale.

2. L'ébauche en matière plastique hybride selon la revendication 1, dans laquelle la section de pièce d'usinage comprend une structure monocouche comprenant la matière plastique hybride à couleur structurelle contenant un colorant.

3. L'ébauche en matière plastique hybride selon la revendication 1, dans laquelle la section de pièce d'usinage comprend une structure multicouche ayant deux ou trois couches, comprenant : une première couche disposée à une extrémité de la structure multicouche et comprenant la matière plastique hybride à couleur structurelle contenant un colorant ; et une seconde couche comprenant une autre matière plastique hybride à couleur structurelle contenant un colorant ayant une couleur d'apparence différente de celle de la matière plastique hybride à couleur structurelle contenant un colorant, une matière plastique hybride à couleur structurelle exempte de colorant, ou une matière plastique hybride différente de ces matières plastiques hybrides.

4. Procédé de fabrication d'une couronne, le procédé comprenant : le fraisage de la section de pièce d'usinage de l'ébauche en matière plastique hybride selon la revendication 1 au moyen d'une technique CAD/CAM basée sur des données de forme tridimensionnelle intraorales d'un patient présentant une dent pilier préparée, afin de fabriquer une couronne destinée à être fixée sur la dent pilier.
